# EUROPEAN PATENT APPLICATION

(11) **EP 3 804 691 A1**
(43) Date of publication of application: **14.04.2021**
(21) Application number: 19812350.7
(22) Date of filing: 09.05.2019
(51) Int. Cl.: A61K 8/81, A61Q 5/12

(54) **HAIR COSMETIC COMPRISING BLOCK COPOLYMER**

(30) Priority: 29.05.2018 JP 2018102647
(71) Applicant: Shiseido Company, Ltd., Chuo-ku Tokyo 104-0061 (JP)
(72) Inventor: MIYAZAWA, Kazuyuki, Tokyo 104-0061 (JP); ITO, Yuji, Tokyo 104-0061 (JP)
(74) Representative: Santarelli
(86) International application number: PCT/JP2019/018614
(87) International publication number: WO 2019/230326

(57) **Abstract**

The invention provides a hair cosmetic comprising a novel block copolymer capable of sustainably imparting properties such as slipperiness to hair.

The hair cosmetic of the present invention comprises a block copolymer having a hydrophobic segment and a hydrophilic segment, wherein the hydrophobic segment has a monomer unit composed of at least one monomer selected from the following Formula 1 and Formula 2, wherein the hydrophilic segment has a monomer unit composed of a monomer of the following formula 3: 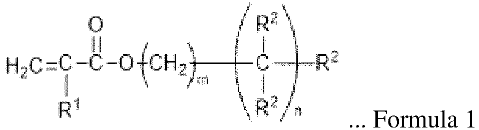 where R¹ is hydrogen or a methyl group, R² is hydrogen or fluorine, m is an integer of 1 to 6, and n is an integer of 5 to 15, 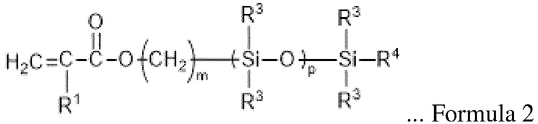 where R¹ is hydrogen or a methyl group, R³ and Rare each independently an alkyl group having 1 to 6 carbon atoms, m is an integer of 1 to 6, and p is an integer of 5 to 70, 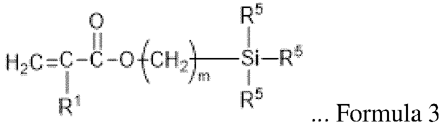 where R¹ is hydrogen or a methyl group, m is an integer of 1 to 6, and R⁵ are each independently a functional group that hydrolyzes and cross-links.

## Description

### FIELD

The present invention relates to a hair cosmetic comprising a novel block copolymer.

### BACKGROUND

Recently, various hair cosmetics using a polymer material or the like have been studied for the purpose of modifying hair and the like.

Patent Literature 1 discloses a hair cosmetic containing (A) 1 to 15% by mass of a vegetable oil, (B) a volatile oil, (C) a high molecular weight dimethylpolysiloxane, and (D) a dimethylpolysiloxane having a hydroxyl group, a polyoxyethylene group, and/or a polyoxypropylene group.

Patent Literature 2 discloses a composition for hair treatment in which a random copolymer having a silyl group to which at least one reactive functional group is bonded is blended.

### [CITATION LIST]

### [PATENT LITERATURE]

[PTL 1] Japanese Unexamined Patent Publication (Kokai) No.2009-221143
[PTL 2] Japanese Unexamined Patent Publication (Kokai) HEI No. 11-302129

### SUMMARY

### [TECHNICAL PROBLEM]

In the field of hair cosmetics, conventionally, a cosmetic which can sustainably impart properties such as slipperiness to hair has been desired.

Accordingly, it is a subject of the present invention to provide a hair cosmetic comprising a novel block copolymer capable of sustainably imparting properties such as slipperiness to hair.

### [SOLUTION TO PROBLEM]

### <Aspect 1>

A hair cosmetic comprising a block copolymer having a hydrophobic segment and a hydrophilic segment,
wherein the hydrophobic segment has a monomer unit composed of at least one monomer selected from the following Formula 1 and Formula 2,
wherein the hydrophilic segment has a monomer unit composed of a monomer of the following formula 3: 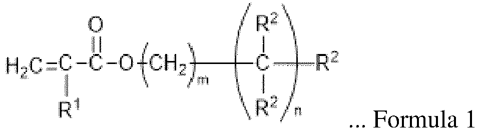
where R¹ is hydrogen or a methyl group,
R² is hydrogen or fluorine,
m is an integer of 1 to 6, and
n is an integer of 5 to 15, 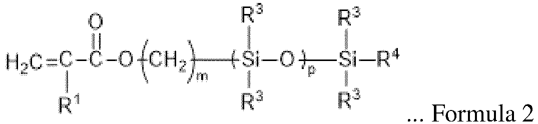
   where R¹ is hydrogen or a methyl group,
   R³ and R⁴ are each independently an alkyl group having 1 to 6 carbon atoms,
   m is an integer of 1 to 6, and
   p is an integer of 5 to 70, 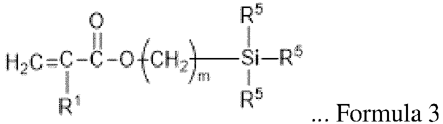
      where R¹ is hydrogen or a methyl group,
      m is an integer of 1 to 6, and
      R⁵ are each independently a functional group that hydrolyzes and cross-links.

### <Aspect 2>

The cosmetic according to aspect 1,
wherein R¹ of Formula 1 is a methyl group, R² is hydrogen or fluorine, m is an integer of 1 to 3, and n is an integer of 10 to 15,
wherein R¹ and R³ of Formula 2 are a methyl group, R⁴ is a butyl group, m is an integer of 1 to 3, p is an integer of 10 to 60, and
wherein R¹ of Formula 3 is a methyl group, R⁵ is at least one selected from a hydrogen atom, an alkoxy group, a halogen atom, an acyloxy group and an amino group, and m is an integer of 1 to 3.

### <Aspect 3>

The cosmetic according to aspect 1 or 2, wherein in the block copolymer, the proportion of the hydrophobic segment of Formula 1 or Formula 2 is from 10 to 90 mol% and the proportion of the hydrophilic segment of Formula 3 is from 5 to 70 mol%.

### <Aspect 4>

The cosmetic according to any one of aspects 1 to 3, wherein the hydrophilic segment further has a monomer unit composed of a monomer of the following Formula 4: 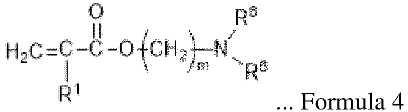
where R¹ is hydrogen or a methyl group,
m is an integer of 1 to 6, and
R⁶ is each independently an alkyl group having 1 to 6 carbon atoms.

### <Aspect 5>

The cosmetic according to aspect 4, wherein in the block copolymer, the proportion of the hydrophilic segment of Formula 4 is 70 mol% or less.

### <Aspect 6>

A hair treatment method comprising applying the cosmetic according to any one of aspects 1 to 5 to hair and crosslinking the functional group that hydrolyzes and cross-links at least on a hair surface.

### [ADVANTAGEOUS EFFECTS OF INVENTION]

According to the present invention, it is possible to provide a hair cosmetic comprising a novel block copolymer capable of sustainably imparting properties such as slipperiness to hair.

### BRIEF DESCRIPTION OF DRAWINGS

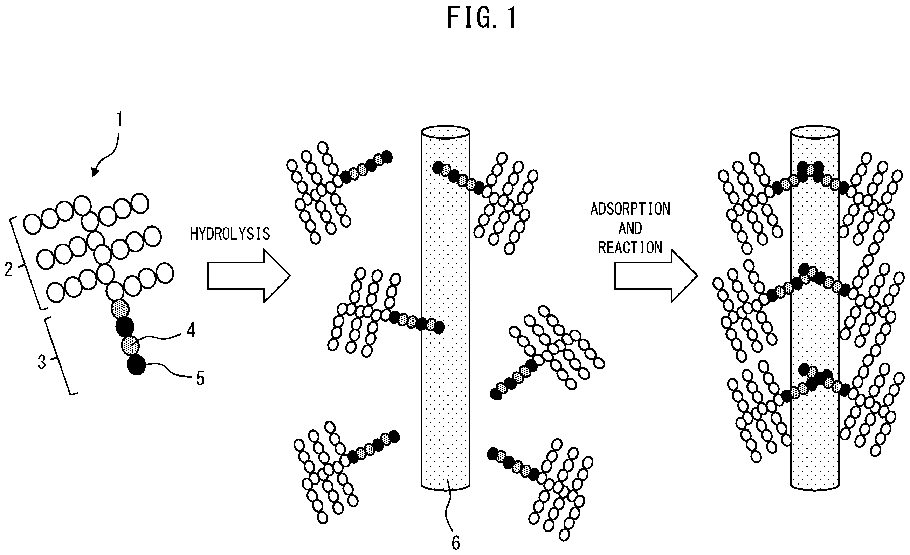

Fig. 1 is a schematic diagram showing a modification mechanism of hair by the block copolymer of one embodiment of the present invention.

### DESCRIPTION OF EMBODIMENTS

Hereinafter, embodiments of the present invention will be described in detail. The present invention is not limited to the following embodiments, and can be variously modified and implemented within the scope of the present invention.

The hair cosmetic of the present invention is a hair cosmetic comprising a block copolymer having a hydrophobic segment and a hydrophilic segment, wherein the hydrophobic segment has a monomer unit composed of at least one monomer selected from the following Formula 1 and Formula 2, and wherein the hydrophilic segment has a monomer unit composed of a monomer of the following Formula 3. 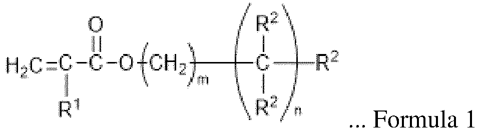
where R¹ is hydrogen or a methyl group,
R² is hydrogen or fluorine,
m is an integer of 1 to 6, and
n is an integer of 5 to 15, 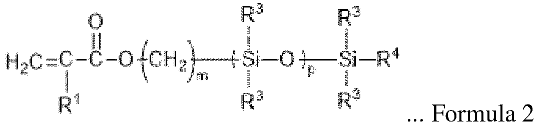
   where R¹ is hydrogen or a methyl group,
   R³ and R⁴ are each independently an alkyl group having 1 to 6 carbon atoms,
   m is an integer of 1 to 6, and
   p is an integer of 5 to 70, 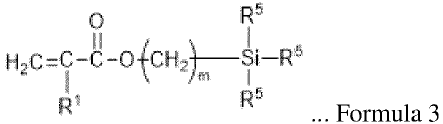
      where R¹ is hydrogen or a methyl group,
      m is an integer of 1 from 6, and
      R⁵ are each independently a functional group that hydrolyzes to crosslink-links.

Although not limited by the principles, it is considered that the operating principle by which such a block copolymer can sustainably impart properties such as slipperiness to the hair is as follows.

For example, when a random copolymer is prepared from a hydrophobic monomer and a hydrophilic monomer, such a copolymer exhibits an intermediate performance such that hydrophobic and hydrophilic properties are mixed together as a whole of a random copolymer because a hydrophobic site and a hydrophilic site are randomly arranged in the copolymer. On the other hand, in the case of a block copolymer, a hydrophobic segment consisting of a hydrophobic monomer and a hydrophilic segment consisting of a hydrophilic monomer are separately formed in the copolymer, so that the block copolymer can be imparted with a portion having different properties such as hydrophobicity and hydrophilicity, respectively.

The block copolymer of the present invention has a particular hydrophobic segment and a specific hydrophilic segment, within which the hydrophilic segment adsorbs and hydrolyzes on the surface of the hair to be surface-treated, as shown in FIG. 1, to form a crosslinked structure at least between the copolymers. Since the copolymer having such a crosslinked structure is easily entangled with hair, it is considered that the copolymer is hardly desorbed from the hair. Further, since the hair has a hydroxyl group on its surface, a functional group in a hydrophilic segment, which is hydrolyzed and subjected to a crosslinking reaction, also reacts with a hydroxyl group on the surface of the hair and binds thereto. As a result, it is considered that the copolymer becomes more difficult to desorb from the hair.

In the case of a random copolymer, since a site to be crosslinked and a site to be hydrophobic are randomly arranged in close proximity, it is considered that, with the formation of a crosslinked structure, the site of hydrophobicity is also entangled in hair at the same time, and as a result, a performance based on the site of hydrophobicity cannot be sufficiently exhibited on hair. On the other hand, in the case of the block copolymer of the present invention, as shown in FIG. 1, hydrophobic segment 2 is arranged separately from hydrophilic segment 3 of the site to be crosslinked, so that the performance based on the hydrophobic segment can be sufficiently exhibited against the hair.

### <<Block copolymer>>

In consideration of the performance and the like based on the hydrophobic segment, the proportion of the hydrophobic segment of Formula 1 or Formula 2 in the copolymer can be set to 10 mol% or more, 15 mol% or more, or 20 mol% or more, and can be set to 90 mol% or less, 80 mol% or less, or 70 mol% or less. In consideration of adsorptivity to hair, formability of a crosslinked structure, and the like, the proportion of the hydrophilic segment of Formula 3 can be set to 5 mol% or more, 10 mol% or more, or 15 mol% or more, and can be set to 70 mol% or less, 60 mol% or less, or 50 mol% or less.

Although there is no particular limitation on the molecular weight of the block copolymer of the present invention, for example, in the gel permeation chromatographic measurement, the number average molecular weight in terms of polystyrene may be in the range of 1000 to 100,000, and is preferably in the range of 2000 to 50,000, and more preferably in the range of 5000 to 20,000. Further, as the molecular weight distribution which is a ratio of the number average molecular weight and the weight average molecular weight, it can be set in a range of 1.05 to 5.0, and is preferably in a range of 1.05 to 3.0.

### <Hydrophobic segment>

### (Monomer of Formula 1)

The following monomer of Formula 1 can constitute a monomer unit of a hydrophobic segment, and can change properties such as hand feeling and the like with respect to hair to which the copolymer is applied:

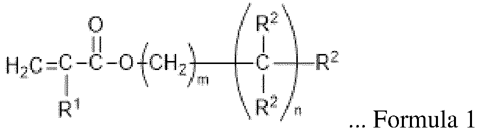

In Formula 1, R¹ is hydrogen or a methyl group, R² is hydrogen or fluorine, m is an integer of 1 to 6, and n is an integer of 5 to 15. From the viewpoint of obtaining properties such as good hand feeling, R¹ of Formula 1 is preferably a methyl group, and R² is preferably hydrogen or fluorine, m is preferably an integer of 1 to 3, and n is preferably an integer of 10 to 15. Here, the sites of (CH₂)ₘ and (CR²₂)ₙ in Formula 1 may be either linear or branched, but is preferably linear.

### (Monomer of Formula 2)

The following monomer of Formula 2 can constitute a monomer unit of a hydrophobic segment, and can improve performance such as slipperiness and fast drying property with respect to hair to which the copolymer is applied:

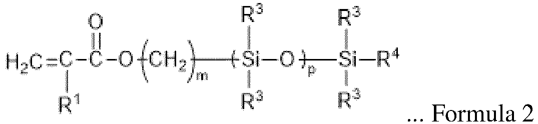

In Formula 2, R¹ is a hydrogen or a methyl group, R³ and R⁴ are each independently an alkyl group having 1 to 6 carbon atoms, m is an integer of 1 to 6, and p is an integer of 5 to 70. From the viewpoint of performance such as slipperiness, fast drying, and the like, R¹ and R³ of Formula 2 are preferably a methyl group, and R⁴ is preferably a butyl group, m is preferably an integer of 1 to 3, p is preferably an integer of 10 to 60, more preferably an integer of 20 to 60, and particularly preferably an integer of 20 to 50. Here, the site of (CH₂)ₘ in Formula 2 may be either linear or branched, but is preferably linear.

### <Hydrophilic segments >

### (Monomer of Formula 3)

The following monomer of Formula 3 constitutes a monomer unit of a hydrophilic segment, and adsorbs and hydrolyzes on the surface of the hair to form a crosslinked structure at least between the copolymers:

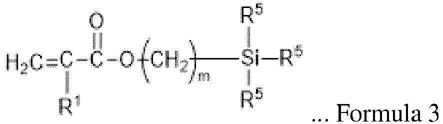

In Formula 3, R¹ is hydrogen or a methyl group, m is an integer of 1 to 6, and R⁵ is each independently a functional group which hydrolyzes and crosslinks. From the viewpoint of adsorptivity to hair, formability of a crosslinked structure, and the like, R¹ of Formula 3 is preferably a methyl group, and R⁵ is preferably at least one selected from a hydrogen atom, an alkoxy group, a halogen atom, an acyloxy group and an amino group, and among them, a methoxy group or an ethoxy group is more preferred, and m is preferably an integer of 1 to 3. Here, the site of (CH₂)ₘ in Formula 3 may be either linear or branched, but is preferably linear.

### <Optional monomer>

The hydrophilic segment of the present invention may further have a monomer unit composed of the following monomer of Formula 4. Since such a monomer unit is ionized to N⁺ and easily adsorbed on the hair, the block copolymer is easily oriented with respect to the hair:

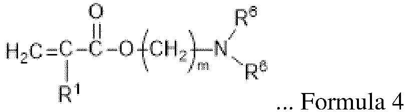

In Formula 4, R¹ is hydrogen or a methyl group, m is an integer of 1 to 6, and R⁶ is each independently an alkyl group having 1 to 6 carbon atoms. From the viewpoint of adsorptivity to hair and the like, R¹ and R⁶ are preferably a methyl group, and m is preferably an integer of 1 to 3. Here, the site of (CH₂)ₘ in Formula 4 may be either linear or branched, but is preferably linear.

In the block copolymer, the proportion of the hydrophilic segment of Formula 4 may be 70 mol% or less, 60 mol% or less, or 50 mol% or less from the viewpoint of adsorptivity to hair and the like.

The block copolymer of the present invention may further have a monomer unit composed of a monomer other than the above Formulas 1 to 4 as long as the effect of the present invention is not impaired. The percentage of such a monomer unit may be in the range of 30 mol% or less, 20 mol% or less, 10 mol% or less, or 5 mol% or less of the total amount of the monomer unit constituting. Examples of such monomers include acrylamide, methacrylamide, methyl acrylamide, methyl methacrylamide, dimethyl methacrylamide, ethyl acrylamide, ethyl methacrylamide, diethyl methacrylamide, N-isopropylacrylamide, N-vinylpyrrolidone, ε-caprolactam, vinyl alcohol, maleic anhydride, diallyldimethylammonium chloride, alkyl acrylate, alkyl methacrylate, N, N'-dimethylacrylamide, styrene, and the like.

### <Method for producing the block copolymer>

The block copolymer of the present invention can be obtained by a known living radical polymerization method. For example, at least one monomer selected from Formula 1 and Formula 2 described above is used to form a hydrophobic segment by a living radical polymerization method, and then the monomer of Formula 3 and, if present, the monomer of Formula 4 may be used to form a hydrophilic segment by a living radical polymerization method to obtain a block copolymer. Alternatively, the monomer of Formula 3 and, if present, the monomer of Formula 4 may be used to form a hydrophilic segment by a living radical polymerization method, and then at least one monomer selected from Formula 1 and Formula 2 may be used to form a hydrophobic segment by a living radical polymerization method to obtain a block copolymer.

The living radical polymerization method is a method in which a catalyst, a chain transfer agent, or the like is added to a conventional radical polymerization method to control the reactivity of a terminal active radical to cause the polymerization to proceed in a pseudo living manner. The molecular weight distribution can be narrowed in comparison with the usual radical polymerization, and the control of the molecular weight is also possible. Specific examples of the known living radical polymerization method include a living radical polymerization method using a non-metal catalyst disclosed in WO 2010/016523 A and the like, a ATRP method by adding a metal complex disclosed in WO 96/030421 A and the like, a TEMPO method for introducing a thermal dissociation group disclosed in US 4,581,429 and the like, a RAFT polymerization method for adding a reversible additional cleavage chain transfer agent disclosed in WO 98/001479 A and the like, and an iniferta method having a photo-thermal dissociation group disclosed in Chem. Express 5 (10), 801 (1990), and the like.

Among them, a living radical polymerization method using a non-metal catalyst which is inexpensive and has little load on the environment is preferred. Such a polymerization method is carried out, for example, using various monomers described above, an initiation compound, a catalyst, a radical polymerization initiator, and optionally a polymerization solvent.

### (Initiation compound)

As the initiation compound, an iodine compound represented by the following Formula 5 is preferably used:

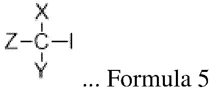

The iodine atom in such an initiation compound is bonded to a secondary or tertiary carbon atom, and X, Y, and Z may be the same or different, and are preferably selected from hydrogen, a hydrocarbon group, a halogen group, a cyano group, an alkoxycarbonyl group, an allyloxycarbonyl group, an acyloxy group, an allyloxy group, an alkoxy group, an alkylcarbonyl group, and an allylcarbonyl group.

It is preferable that the iodine atom is bonded to a secondary or tertiary carbon atom in consideration of the dissociation property of iodine. As a result, at least 2 of X, Y and Z are not hydrogen atoms. Specific examples of X, Y and Z will be described below, but are not limited thereto.

Examples of the hydrocarbon group include an alkyl group, an alkenyl group, an alkynyl group, an aryl group, and an arylalkyl group. Specifically, examples thereof include alkyl groups such as methyl, ethyl, propyl, isopropyl, butyl, isobutyl, 2-methylpropyl, t-butyl, pentyl, dodecyl; alkenyl groups including double bond such as vinyl, allyl, 2-methylvinyl, butenyl, butadienyl; alkynyl groups including triple bond such as acetylene, methylacetylene; aryl groups such as phenyl, naphthyl, methylphenyl, ethylphenyl, propylphenyl, dodecylphenyl, biphenyl, in which the aryl group can include heterocyclic rings such as pyridinyl, imidazolinyl; arylalkyl groups such as phenylmethyl, 1-phenylethyl, 2-phenylethyl, 2-phenylpropyl, and the like.

Examples of the halogen group include a fluorine atom, a chlorine atom, a bromine atom, and an iodine atom.

Examples of the alkoxycarbonyl group or allyloxycarbonyl group include methoxycarbonyl, ethoxycarbonyl, propylcarbonyl, cyclohexylcarbonyl, benzyloxycarbonyl, phenoxycarbonyl, and naphthoxycarbonyl.

Examples of the acyloxy group or the allyloxy group include acetoxy, ethylcarbonyloxy, cyclohexylcarbonyloxy, benzoyloxy, and naphthylcarboxyoxy.

Examples of the alkoxy group include methoxy, ethoxy, methoxyethoxy, and phenoxyethoxy.

Examples of the alkylcarbonyl group or allylcarbonyl group include methylcarbonyl, ethylcarbonyl, and phenylcarbonyl.

Preferred specific examples of the initiation compound include 1-iodo-1-phenylethane, 2-iodo-2-cyanopropane, 2-iodo-2-cyano-4-methylpentane.

In addition, in such a polymerization method, the molecular weight of the copolymer can be controlled by the amount of the initiation compound. By setting the number of moles of the monomer relative to the number of moles of the initiation compound, it is possible to control an arbitrary molecular weight or a large or small molecular weight. The molar ratio of the initiation compound to the monomer is not particularly limited and can be specified appropriately depending on the molecular weight and the like required. For example, when 1 mol of an initiation compound is used to polymerize using 500 mol of a monomer having a molecular weight of 100, a theoretical molecular weight of 1 × 100 × 500=50,000 can be provided.

### (Catalyst)

As the catalyst, a nonmetallic compound which becomes a radical capable of abstracting an iodine of an initiation compound, or iodine at a copolymer terminal, for example, a phosphorus compound, a nitrogen compound or an oxygen compound, or the like, having such a property, can be used.

Examples of the phosphorus compound include, but are not limited to the following, phosphorus halide containing an iodine atom, a phosphite-based compound, a phosphinate-based compound, and the like, and examples of the nitrogen compound include an imide-based compound, hydantoins, barbituric acids, and cyanuric acids, and examples of the oxygen compound include a phenol-based compound, an iodooxyphenyl compound, and vitamins. These may be used alone or in combination of 2 or more thereof.

Specifically, examples of the phosphorus compound include a halogenated phosphorus containing an iodine atom, a phosphite-based compound, or a phosphinate-based compound, and for example, phosphorus dichloroiodide, phosphorus dibromoiodide, phosphorus triiodide, dimethylphosphite, diethylphosphite, dibutylphosphite, di (perfluoroethyl) phosphinate, diphenylphosphite, dibenzylphosphite, bis (2-ethylhexyl) phosphite, bis (2,2,2-trifluoroethyl) phosphite, diallylphosphite, ethylene phosphite, ethylphenylphosphinate, phenylphenylphosphinate, ethylmethylphosphinate, phenylmethylphosphinate, and the like.

Examples of the nitrogen compound include imides such as succinimide, 2,2-dimethylsuccinimide, α,α-dimethyl-β-methylsuccinimide, 3-ethyl-3-methyl-2,5-pyrrolidinedione, cis-1,2,3,6-tetrahydrophthalimide, α-methyl-α-propylsuccinimide, 5-methylhexahydroisoindole-1,3-dione, 2-phenylsuccinimide, α-methyl-α-phenylsuccinimide, 2,3-diacetoxysuccinimide, maleimide, phthalimide, 4-methylphthalimide, N-chlorophthalimide, N-bromophthalimide, 4-nitrophthalimide, 2,3-naphthalenecarboxyimide, pyromellitodiimide, 5-bromoisoindole-1,3-dione, N-chlorosuccinimide, N-bromosuccinimide, N-iodosuccinimide, and the like. Examples of the hydantoins include hydantoin, 1-methylhydantoin, 5,5-dimethylhydantoin, 5-phenylhydantoin, 1,3-diiodo-5,5-dimethylhydantoin, and the like. Examples of the barbituric acids include barbituric acid, 5-methylbarbituric acid, 5,5-diethylbarbituric acid, 5-isopropylbarbituric acid, 5,5-dibutylbarbituric acid, thiobarbituric acid, and the like. Examples of the cyanuric acids include cyanuric acid, N-methylcyanuric acid, triiodocyanuric acid, and the like.

Examples of the oxygen compound include a phenol-based compound which has a phenolic hydroxyl group with a hydroxyl group at an aromatic ring, an iodooxyphenyl compound which is an iodide of the phenolic hydroxyl group thereof, and vitamins. Examples of the phenols include phenol, hydroquinone, 4-methoxyphenol, 4-t-butylphenol, 4-t-butyl-2-methylphenol, 2-t-butyl-4-methylphenol, catechol, resorcin, 2,6-di-t-butyl-4-methylphenol, 2,6-dimethylphenol, 2,4,6-trimethylphenol, 2,6-di-t-butyl-4-methoxyphenol, polymeric microparticles supported with a polymer that is polymerized with 4-hydroxystyrene, or supported with its hydroxyphenyl group, a monomer having a phenolic hydroxyl group such as methacrylic acid 3,5-di-t-butyl-4-hydroxyphenylethyl. Since these are added as polymerization inhibitors in an ethylenically unsaturated monomer, it is also possible to exert an effect by using an ethylenically unsaturated monomer of a commercially available product as it is without purification. Examples of the iodooxyphenyl compound include thymol iodide and the like, and examples of the vitamins include vitamin C and vitamin E.

The amount of the catalyst to be used is generally less than the number of moles of the radical polymerization initiator, and may be arbitrarily determined in consideration of the control state of the polymerization and the like.

### (Radical polymerization initiator)

As the radical polymerization initiator, conventionally known ones can be used, and there is no particular limitation, and for example, an organic peroxide or an azo compound or the like can be used. Specifically, examples thereof include benzoyl peroxide, dicumyl peroxide, diisopropyl peroxide, di-t-butyl peroxide, t-butyl peroxybenzoate, t-hexyl peroxybenzoate, t-butyl peroxy-2-ethylhexanoate, t-hexylperoxy-2-ethylhexanoate, 1,1-bis(t-butyl peroxy)3,3,5-trimethylcyclohexane, 2,5-dimethyl-2,5-bis(t-butylperoxy)hexyl-3,3-isopropylhydroperoxide, t-butyl hydroperoxide, dicumyl hydroperoxide, acetyl peroxide, bis(4-t-butylcyclohexyl)peroxydicarbonate, isobutyl peroxide, 3,3,5-trimethylhexanoylperoxide, lauryl peroxide, 1,1-bis(t-butylperoxy)3,3,5-trimethylcyclohexane, 1,1-bis(t-hexylperoxy)3,3,5-trimethylcyclohexane, 2,2'-azobis(isobutyronitrile), 2,2'-azobis(2,4-dimethylvaleronitrile), 2,2'-azobis(4-methoxy-2,4-dimethylvaleronitrile), dimethyl 2,2'-azobis(isobutyrate), and the like.

From the viewpoint of polymerizability and the like, the radical polymerization initiator can be used in a range of 0.001 mol times or more, 0.002 mol times or more, or 0.005 mol times or more, based on the number of moles of the monomer, and can be used in a range of 0.1 mol times or less, 0.05 mol times or less, or 0.01 mol or less.

### (Polymerization solvent)

If necessary, a polymerization solvent can be used. As such a polymerization solvent, a solvent which does not exhibit reactivity with respect to the functional group of the monomer is appropriately selected. Examples thereof include, but not limited to the following, hydrocarbonic solvents such as hexane, octane, decane, isodecane, cyclohexane, methylcyclohexane, toluene, xylene, ethylbenzene, cumene; alcoholic solvents such as methanol, ethanol, propanol, isopropanol, butanol, isobutanol, hexanol, benzyl alcohol, cyclohexanol; hydroxyl-containing glycol ethers such as ethylene glycol, diethylene glycol, propylene glycol, dipropylene glycol, methyl cellosolve, ethyl cellosolve, butyl cellosolve, propylene glycol monomethyl ether, propylene glycol monoethyl ether, propylene glycol propyl ether, butylcarbitol, butyltriethylene glycol, methyldipropylene glycol; glycolic solvents such as diglyme, triglyme, methyl cellosolve acetate, propylene glycol monomethyl ether acetate, dipropylene glycol butyl ether acetate, diethylene glycol monobutyl ether acetate; etheric solvents such as diethyl ether, dimethyl ether, dipropyl ether, methylcyclopropyl ether, tetrahydrofuran, dioxane, anisole; ketonic solvents such as dimethyl ketone, diethyl ketone, ethyl methyl ketone, isobutyl methyl ketone, cyclohexanone, isophorone, acetophenone; ester solvents such as methyl acetate, ethyl acetate, butyl acetate, propyl acetate, methyl butyrate, ethyl butyrate , caprolactone, methyl lactate, ethyl lactate; halogenated solvents such as chloroform, dichloromethane, dichloroethane, o-dichlorobenzene; amide-based solvents such as formamide, N,N-dimethylformamide, N,N-dimethylacetamide, 2-pyrrolidone, N-methyl-2-pyrrolidone, ε-caprolactam; dimethyl sulfoxide, sulfolane, tetramethylurea, ethylene carbonate, propylene carbonate, dimethyl carbonate, diethyl carbonate, nitromethane, acetonitrile, nitrobenzene, dioctylphthalate, and the like.

### (Polymerization temperature)

The polymerization temperature is appropriately adjusted by the half-life of the radical polymerization initiator, and is not particularly limited, and may be, for example, 0 °C or more or 30 °C or more, and may be 150 °C or less or 120 °C or less.

### (Polymerization time)

It is preferable to continue polymerization until the monomer is gone, and the polymerization time is not particularly limited, and may be, for example, 0.5 hours or more, 1 hours or more, or 2 hours or more, and may be 48 hours or less, 24 hours or less, or 12 hours or less.

### (Polymerization atmosphere)

The polymerization atmosphere is not particularly limited, and may be polymerized as it is under an atmospheric atmosphere, that is, oxygen may be present within a normal range in the polymerization system, or may be carried out under a nitrogen atmosphere in order to remove oxygen if necessary. Impurities may be removed from various materials to be used by distillation, activated carbon, alumina, or the like, but a commercially available product may be used as it is. Also, the polymerization may be carried out under light shielding and may be carried out in a transparent container such as glass.

### <<Hair cosmetics>>

The block copolymer of the present invention can be used as a hair cosmetic, for example, by dissolving or dispersing in a nonaqueous solvent.

### (Solvent)

When the hair cosmetic is a nonaqueous composition, as a solvent, an aliphatic hydrocarbon, an aromatic hydrocarbon, a chlorine compound hydrocarbon, an ether-based solvent, an alcohol-based solvent such as an aliphatic 1 to 4 valent alcohol having 1 to 4 carbon atoms, a cellosolve-based solvent such as ethyl cellosolve, butyl cellosolve, dioxane, methyl acetate, diformamide, or the like can be used. Among them, methanol, ethanol, isopropanol, propylene glycol, and the like, which are aliphatic 1 to 2 valent alcohols, are preferred, and ethanol and isopropanol are more preferred from the viewpoint of safety.

When the hair cosmetic is an aqueous composition, water such as ion-exchanged water or distilled water can be used as a solvent.

### (Blending amount)

The amount of the block copolymer to be blended in the hair cosmetic is not particularly limited, but may be, for example, 0.1% by mass or more, 0.2% by mass or more, or 0.3% by mass or more based on the total amount of the cosmetic, and may be 50% by mass or less, 25% by mass or less, or 10% by mass or less, preferably in a range of 0.5% by mass to 3% by mass.

### (Dosage form)

The dosage form of the hair cosmetic is not particularly limited, and any dosage form may be used as long as it can exhibit the effect of the present invention. Examples thereof include liquid, emulsion, creamy, gel, mist, spray, aerosol, mousse, and the like.

### (Optional components)

In the hair cosmetic of the present invention, various components can be appropriately blended within a range that does not affect the effect of the present invention. Various components may be additive components which can usually be blended into cosmetics, for example, liquid fats, solid fats, waxes, oils such as higher fatty acids, rosehip oils, tsubaki oils, higher alcohols, anionic surfactants, cationic surfactants, amphoteric surfactants, nonionic surfactants, moisturizers, water-soluble polymers, thickeners, film agents such as silicone modified polysaccharide, sequestering agents, lower alcohols, polyhydric alcohols, various extracts, sugars, amino acids, organic amines, polymer emulsions, chelating agents, ultraviolet absorbers, pH adjusting agents, skin nutritional agents, royal jelly extracts, vitamins, pharmaceuticals, quasi-drugs, water-soluble drugs applicable to cosmetics or the like, antioxidants, buffers, preservatives, antioxidant auxiliary agents, propellants, organic-based powders, pigments, dyes, colorants, perfumes, water, acid components, alkali components, and the like. Here, water, acid, and alkali are preferably set as separate agents from the block copolymer.

### <Hair treatment method>

As a method for treating hair by the hair cosmetic of the present invention, such a cosmetic is applied to hair, and a functional group, which is hydrolyzed and subjected to a crosslinking reaction, in a block copolymer contained in the cosmetic is crosslinked to each other at least on a hair surface. Examples of the crosslinking method include a method utilizing a reaction with water, an acid or an alkali, a reaction with heat, and the like. Specifically, there is a method in which, using a known means such as coating, the hair cosmetic of the present invention is applied to hair, and then such a hair can be hydrolyzed and cross-linked by bringing the hair into contact with water, steam, acid or alkali, or by heating the hair. Alternatively, the hair previously treated with water, acid or alkali may be subjected to hydrolyse and crosslinking by applying the hair cosmetic.

In addition, a method in which water, an acid or an alkali is mixed to the hair cosmetic of the present invention and immediately applied is also conceivable, but it is preferable that the hair cosmetic and the water, the acid or the alkali be applied to the hair separately. The reaction with water, an acid or an alkali usually proceeds even at room temperature, but may be heated to accelerate the reaction. When the progression of the crosslinking reaction may be slow, it is also possible to naturally crosslink only by moisture in the atmosphere. In addition, an instrument such as a brush, or a comb can be appropriately used as necessary for uniformly applying and treating in any case.

The acid and alkali used in the hair treatment method of the present invention are not particularly limited as long as it can proceed the reaction of the functional group in the block copolymer, which is hydrolyzed and subjected to the crosslinking reaction, , and organic or inorganic acids or alkalis can be used. Each of these acids and alkalis may be used alone or in a mixture of 2 or more thereof, and may be a mixture with water.

### EXAMPLES

Hereinafter, the present invention will be described in more detail with reference to Examples, but the present invention is not limited thereto. Note that, hereinafter, unless otherwise specified, the blending amount is shown in parts by mass.

### <<Examples 1 to 5 and Comparative Examples 1 to 2>>

### (Synthesis of copolymers)

### (Copolymer 1)

25 parts by mass of a monomer of the following Formula 2, 0.167 parts by mass of iodine, 0.3 parts by mass of 2,2'-azobis (isobutyronitrile), and 0.082 parts by mass of N-iodosuccinimide were charged into a reaction vessel equipped with a stirrer, a reflux condenser, a thermometer, and a nitrogen inlet tube, and the mixture was polymerized at 60 °C for 2 hours while nitrogen bubbling to prepare a hydrophobic segment portion. 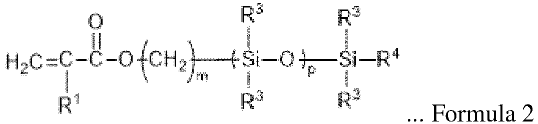 where R¹ and R³ are a methyl group, R⁴ is butyl group, m is 3, and p is 10.

Subsequently, 11.7 parts by mass of a monomer of the following Formula 3 and 6.4 parts by mass of a monomer of the following Formula 4 were added, and further polymerized at the same temperature for 2 hours to form a hydrophilic block portion, thereby obtaining a block copolymer having a hydrophobic segment and a hydrophilic segment. From the amount of each monomer raw material to be used, when the molar ratio of the obtained block copolymer was converted, the proportion of the monomer unit of Formula 2 was about 49.4 mol%, the proportion of the monomer unit of Formula 3 was about 25.2 mol%, and the proportion of the monomer unit of Formula 4 was about 25.4 mol%. 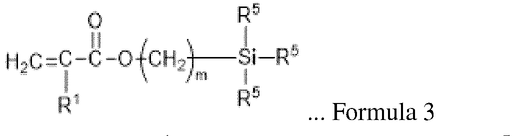 where R¹ is a methyl group, m is 3, and R⁵ is an ethoxy group. 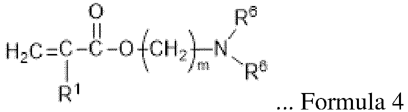 where R¹ and R⁶ are a methyl group, and m is 2.

### (Copolymer 2)

The block copolymer of copolymer 2 was prepared in the same manner as in copolymer 1, except that the amount of each monomer added was 30 parts by mass of the monomer of Formula 2 above, 21 parts by mass of the monomer of Formula 3 above, and 0 parts by mass of the monomer of Formula 4 above.

### (Copolymer 3)

The block copolymer of copolymer 3 was prepared in the same manner as in copolymer 1, except that p of the monomer of Formula 2 above was changed to 60, and the amount of each monomer added was changed to 45 parts by mass of the monomer of Formula 2 above, 3.5 parts by mass of the monomer of Formula 3 above, and 1.9 parts by mass of the monomer of Formula 4 above.

### (Copolymer 4)

The block copolymer of copolymer 4 was prepared in the same manner as in copolymer 1, except that p of the monomer of Formula 2 above was changed to 30, and the amount of each monomer added was changed to 45 parts by mass of the monomer of Formula 2 above, 5.5 parts by mass of the monomer of Formula 3 above, and 0 parts by mass of the monomer of Formula 4 above.

### (Copolymer 5)

The block copolymer of copolymer 5 was prepared in the same manner as in copolymer 1, except that p of the monomer of Formula 2 above was changed to 30, and the amount of each monomer added was changed to 45 parts by mass of the monomer of Formula 2 above, 2.8 parts by mass of the monomer of Formula 3 above, and 0 parts by mass of the monomer of Formula 4 above.

### (Copolymer 6)

A random copolymer was prepared by charging 25 parts by mass of the monomer of the above Formula 2, 11.7 parts by mass of the monomer of the above Formula 3, 6.3 parts by mass of the monomer of the above Formula 4, and 0.0082 parts by mass of N-iodosuccinimide into a reaction vessel equipped with a stirrer, a reflux condenser, a thermometer, and a nitrogen inlet tube, and polymerizing the mixture at 80 °C for 2 hours while bubbling with nitrogen.

### (Copolymer 7)

After dissolving 8.0g (27mmol) of 3-methacryloxypropyltriethoxysilane and 4.0g (40mmol) of methyl methacrylate in 100ml of ethanol and heating and stirring under a stream of nitrogen at 70 °C for 1 hours, 0.05g of potassium persulfate was added and reacted overnight to complete the copolymerization reaction. The reaction solution was cooled to room temperature and concentrated by decompression. The residue was dissolved in 10ml of ethanol and added in 500ml of n-hexan. The precipitate was fractionated and the desired random copolymer was obtained. In the obtained random copolymer, the proportion of the 3-methacryloxypropyltriethoxysilane monomer unit corresponding to y was 40.3 mol%, and the proportion of the methyl methacrylate monomer unit was 59.7 mol%.

### <Preparation method of cosmetics and evaluation samples>

Cosmetics were prepared by collecting 3 parts by mass of each of copolymers 1 to 7 and dissolving them in 97 parts by mass of ethanol, and filling each of them into a spray container.

Subsequently, the hair was immersed in a wetting liquid containing citric acid for 5 minutes, and then the hair was taken out from the wetting liquid, and about 0.5g of the cosmetic was sprayed on the hair. The hair was blended with the cosmetic through a comb and left in the air for 5 minutes and then the hair was dried with a hair dryer. Then, a hair iron of 180 °C was applied to the hair for 15 seconds and passed through a shampoo and a conditioner to prepare evaluation samples.

### <Evaluation method>

### (Evaluation of slipperiness)

Sensory evaluation for slipperiness by 20 expert panels was performed. As an evaluation method, a questionnaire was conducted on slipperiness on hair not coated with the cosmetic, hair immediately after production, and hair repeating a shampoo and a conditioner 30 times each, and evaluated according to the following criteria. The results are shown in Table 1.

A: More than 16 out of 20 respondents responded that slipperiness was good.
B: 12 to 15 out of 20 respondents responded that slipperiness was good.
C: 6 to 11 out of 20 respondents responded that slipperiness was good.
D: 0 to 5 out of 20 respondents responded that slipperiness was good.

### (Fast drying evaluation)

Sensory evaluation for fast drying by 20 expert panels was performed. As an evaluation method, a questionnaire was conducted on the degree of dryness of hair, in which hair immediately after production and hair repeating the shampoo and the conditioner 30 times each were immersed in water for 1 minutes, taken out, and left in an atmosphere for 10 minutes after towel drying, and evaluated by the following criteria. The results are shown in Table 1.

A: More than 16 out of 20 respondents responded that hair was dry.
B: 12 to 5 out of 20 respondents responded that hair was dry.
C: 6 to 11 out of 20 respondents responded that hair was dry.
D: 0 to 5 out of 20 respondents responded that hair was dry.

**[Table 1]**

| | Copolymer | | p of Formula 2 | Slipperiness | | Fast drying | |
|---|---|---|---|---|---|---|---|
| | | | | Immediately after | After 30 times | Immediately after | After 30 times |
| Example 1 | 1 | Block | 10 | C | C | B | B |
| Example 2 | 2 | Block | 10 | C | C | C | C |
| Example 3 | 3 | Block | 60 | A | A | A | A |
| Example 4 | 4 | Block | 30 | B | B | B | B |
| Example 5 | 5 | Block | 30 | B | B | A | A |
| Comparative Example 1 | 6 | Random | 10 | B | D | B | D |
| Comparative Example 2 | 7 | Random | (0)^{x} | D | D | D | D |

| | | | | | | | |
|---|---|---|---|---|---|---|---|
| x: Numbers of (Si-O) sites in methyl methacrylate monomer. | | | | | | | |

### <Results>

As is apparent from Table 1, it could be confirmed that cosmetics containing the block copolymer of Examples 1 to 5 can sustainably impart slipperiness as compared with cosmetics containing the random copolymer of Comparative Examples 1 to 2. In addition, it has been found that as the number of p of the monomer of Formula 2 or the proportion of the monomer unit of Formula 2 increases, a fast drying property can also be imparted in a sustained manner.

### <<Formulation example of hair cosmetics>>

Hereinafter, the formulation example of the hair cosmetic of the present invention will be described, but the present invention is not limited to this illustration. Note that the hair coating agent described in the following formulation example can sustainably impart a feeling of use such as slipperiness to hair, and also has a fast drying property.

### <Formulation Example 1 Hair coating agent>

| (Component) | (% by mass) |
|---|---|
| Copolymer 1 | 3 |
| Ethanol | 90 |
| Dimethicone | 3 |
| Glycerin | 1 |
| Perfume | q.s. |

### (Method for producing a hair coating agent)

The block copolymer was added to a vessel containing ethanol while stirring to dissolve. Then, dimethicone, glycerin, and perfume were added to the container while stirring to prepare a hair coat agent.

### REFERENCE SIGNS LIST

- 1: Block copolymer
- 2: Hydrophobic segment
- 3: Hydrophilic segment
- 4: Adsorbable monomer units
- 5: Crosslink-linking reactive monomer units
- 6: Hair

## Claims

1. A hair cosmetic comprising a block copolymer having a hydrophobic segment and a hydrophilic segment,
wherein the hydrophobic segment has a monomer unit composed of at least one monomer selected from the following Formula 1 and Formula 2,
wherein the hydrophilic segment has a monomer unit composed of a monomer of the following formula 3: 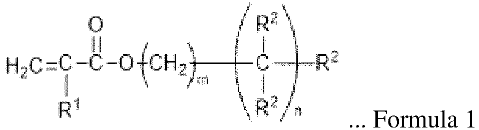
where R¹ is hydrogen or a methyl group,
R² is hydrogen or fluorine,
m is an integer of 1 to 6, and
n is an integer of 5 to 15, 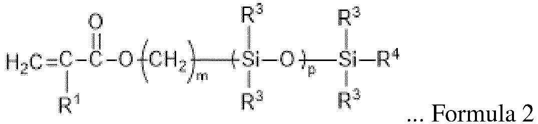
where R¹ is hydrogen or a methyl group,
R³ and R⁴ are each independently an alkyl group having 1 to 6 carbon atoms,
m is an integer of 1 to 6, and
p is an integer of 5 to 70, 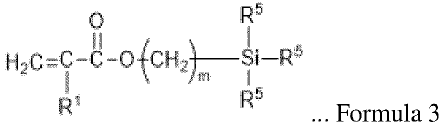
where R¹ is hydrogen or a methyl group,
m is an integer of 1 to 6, and
R⁵ are each independently a functional group that hydrolyzes and cross-links.

2. The cosmetic according to claim 1,
wherein R¹ of Formula 1 is a methyl group, R² is hydrogen or fluorine, m is an integer of 1 to 3, and n is an integer of 10 to 15,
wherein R¹ and R³ of Formula 2 are a methyl group, R⁴ is a butyl group, m is an integer of 1 to 3, p is an integer of 10 to 60, and
wherein R¹ of Formula 3 is a methyl group, R⁵ is at least one selected from a hydrogen atom, an alkoxy group, a halogen atom, an acyloxy group and an amino group, and m is an integer of 1 to 3.

3. The cosmetic according to claim 1 or 2, wherein in the block copolymer, the proportion of the hydrophobic segment of Formula 1 or Formula 2 is from 10 to 90 mol% and the proportion of the hydrophilic segment of Formula 3 is from 5 to 70 mol%.

4. The cosmetic according to any one of claims 1 to 3, wherein the hydrophilic segment further has a monomer unit composed of a monomer of the following Formula 4: 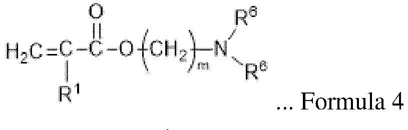
where R¹ is hydrogen or a methyl group,
m is an integer of 1 to 6, and
R⁶ is each independently an alkyl group having 1 to 6 carbon atoms.

5. The cosmetic according to claim 4, wherein in the block copolymer, the proportion of the hydrophilic segment of Formula 4 is 70 mol% or less.

6. A hair treatment method comprising applying the cosmetic according to any one of claims 1 to 5 to hair and crosslinking the functional group that hydrolyzes and cross-links at least on a hair surface.
